# EUROPEAN PATENT APPLICATION

(11) **EP 3 042 602 A1**
(43) Date of publication of application: **13.07.2016**
(21) Application number: 15195352.8
(22) Date of filing: 19.11.2015
(51) Int. Cl.: A61B 5/00, A61B 5/1455, H02J 7/02, H02J 9/00, A61N 1/372

(54) **BIOLOGICAL INFORMATION MEASURING DEVICE AND BIOLOGICAL INFORMATION MEASURING METHOD**

(30) Priority: 28.11.2014 JP 2014241444
(71) Applicant: Konica Minolta, Inc., Tokyo 100-7015 (JP)
(72) Inventor: Tateda, Norihiro, Tokyo, 100-7015 (JP); Kawada, Kenji, Tokyo, 100-7015 (JP); Kameda, Masanobu, Tokyo, 100-7015 (JP)
(74) Representative: Hoffmann Eitle

(57) **Abstract**

A biological information measuring device controls a measuring operation of a measuring section for measuring biological information to be suspended during the time when a battery section is charged by a charging power supply section in a noncontact manner. A biological information measuring method using a biological information measuring device allows one of a measuring step of measuring biological information and a charging step of performing charging of electric power in a noncontact manner to be selectively executed.

## Description

### Technical Field

The present invention relates to a biological information measuring device and a biological information measuring method for measuring biological information.

### Background Art

Various types of biological information measuring devices for measuring predetermined physiological information (biological information) on living bodies are known in order to determine conditions of the living bodies. For example, a sphygmograph measures pulse waves of a living body. A pulse oximeter measures a blood oxygen saturation level and a heart rate of a living body, for example. A cardiac output flowmeter measures a cardiac output. A blood glucose meter measures a blood glucose level of a living body, for example. To measure such biological information, a photoelectric technique is employed as one of techniques for measuring biological information. A biological information measuring device using the photoelectric technique measures biological information by, for example, illuminating a finger of a living body and receiving light therefrom.

Such a biological information measuring device is disclosed in, for example, Japanese Unexamined Patent Publication No. 2012-55593 (Patent Document 1). The biological information measuring system disclosed in Patent Document 1 comprises: a plurality of living body measurement blocks each including a measuring section that measures a living body and outputs measured information, a storage part that stores a measured information output from the measuring section and measuring section specifying information specifying the measuring section, and a transmission part that transmits the measured information and the measuring section specifying information to an external device; and a processing block having a reception part that receives the measured information and the measuring section specifying information from the transmission part of each of the plurality of living body measurement blocks, and a processing part that processes the measured information as specified living body information based on a correlation between the measuring section specifying information and information specifying a living body irrespective of which living body measurement block has transmitted the measured information. In the biological information measuring system, the living body measurement block includes a power source section having a battery for supplying electric power to each component described above, the processing block includes a charging section for charging the battery, and the charting section includes a noncontact charging induction portion.

A secondary battery used in a biological information measuring device has a relatively small charge capacity. Therefore, when charging the secondary battery in a wired way, the device is unlikely to generate a large amount of heat because of a small current amount. Thus, the heat generation during charging will not cause a significant problem. In the case of charging using a USB, for example, the current amount is about 500 mA at a voltage of 5 V, and the device does not generate a large amount of heat. On the other hand, in the case of charging a secondary battery in a noncontact manner (a wireless charging system or a noncontact power transfer system) as in the noncontact charging induction portion of Patent Document 1, a circuit for performing noncontact charging is likely to generate heat. Some Safety Standards concerning biological information measuring devices define that a device should not exceed a predetermined temperature during measurement. For example, the IEC 60601-1 series defines that the portion which comes into contact with a patient should not exceed 41°C during measurement.

Here, IEC stands for International Electrotechnical Commission. The IEC60601 series is a series of technical standards for safety of medical electrical equipment, and generally classified into two parts: the IEC 60601-1 series, which is basic standards, and the IEC60601-2 series, which is individual standards. At the current year of 2014, the third edition (Ed.3) thereof takes effect.

### Summary of Invention

The present invention has been made in view of the foregoing problems, and has an object of providing a biological information measuring device and a biological information measuring method that can satisfy predetermined safety standards concerning biological information measuring devices even in the case of employing noncontact charging manner.

A biological information measuring device according to the present invention performs control to stop a measuring operation of a measuring section for measuring biological information during charging of a battery section by a charging power supply section that charges the battery section in a noncontact manner. In a biological information measuring method using a biological information measuring device according to the present invention, one of a measuring step of measuring biological information and a charging step of performing charging of electric power in a noncontact manner is selectively executed. Thus, the biological information measuring device can satisfy predetermined safety standards concerning biological information measuring devices.

These and other objects, features, and advantages of the present invention will become more apparent upon reading the following detailed description along with the accompanying drawings.

### Brief Description of Drawings

FIG. 1 is a block diagram showing a configuration of a biological information measuring device and a charger for the biological information measuring device according to an embodiment of the present invention.
FIG. 2A is a perspective view illustrating a configuration of the biological information measuring device.
FIG. 2B is a cross-sectional view showing the configuration of the biological information measuring device.
FIG. 3 illustrates a state where the biological information measuring device is mounted on the charger.
FIG. 4 is a flowchart showing an operation of the biological information measuring device that starts charging during the time when measurement is performed.
FIG. 5 is a flowchart showing an operation of the biological information measuring device that starts charging when no measurement is performed.

### Description of Embodiments

An embodiment of the present invention will be described with reference to the drawings. The same components are designated by the same reference characters, and description thereof is not repeated when unnecessary. Numerals having suffixes herein identify general parts, and numerals having suffixes identify specific components.

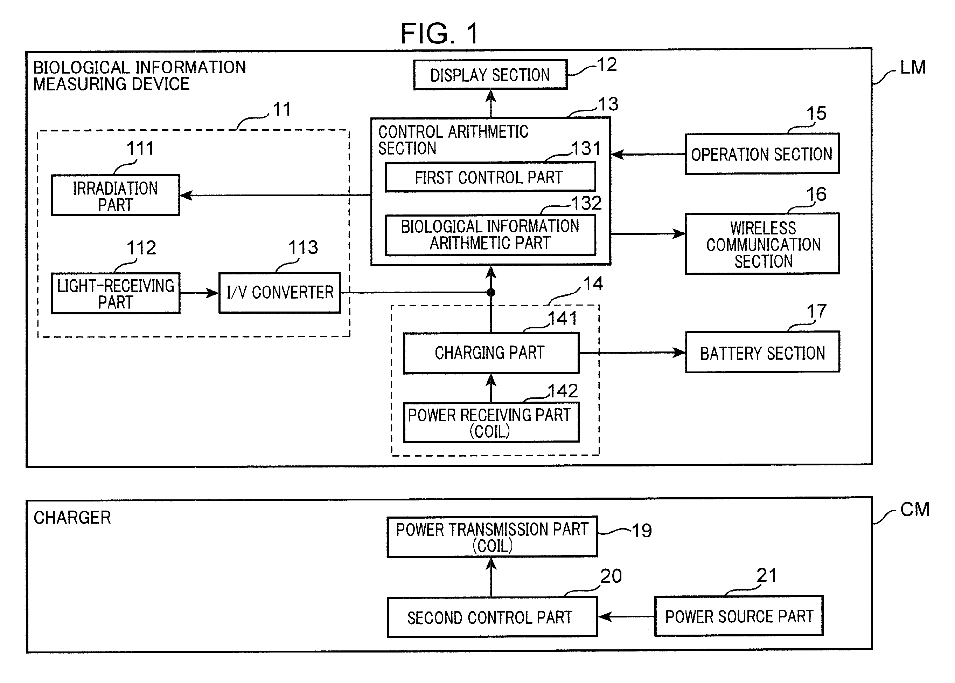

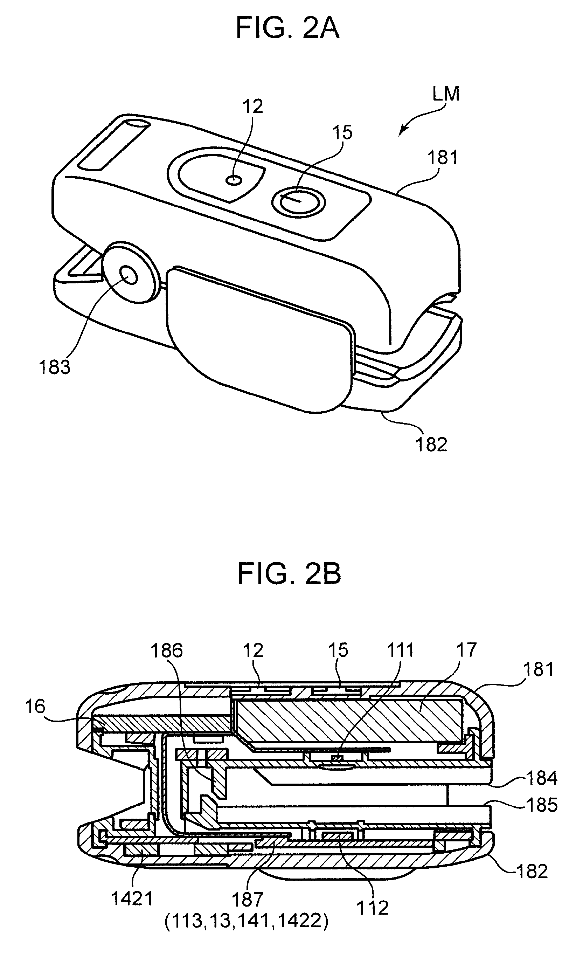

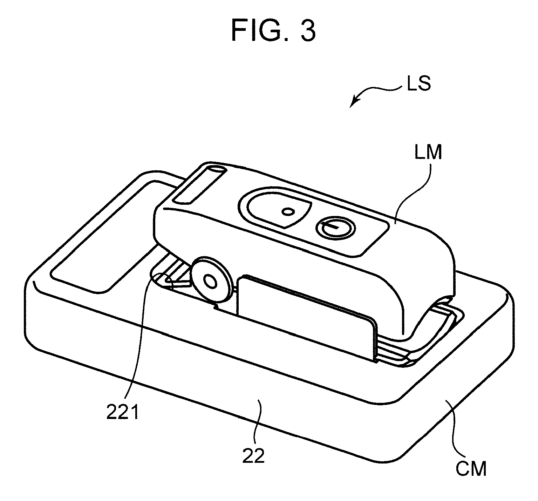

FIG. 1 is a block diagram showing a configuration of a biological information measuring device and a charger for the biological information measuring device according to an embodiment. FIGS. 2A and 2B illustrate the configuration of the biological information measuring device according to the embodiment. FIG. 2A is a perspective view illustrating an appearance of the biological information measuring device, and FIG. 2B is a cross-sectional view of the biological information measuring device. FIG. 3 illustrates a state of the biological information measuring device which is mounted on the charger.

The biological information measuring device according to the embodiment is a device for measuring predetermined physiological information (biological information) from a living body when being placed with a part of the living body. More specifically, in the present embodiment, a biological information measuring device LM is a photoelectric device that is put on a finger (e.g., a maniphalanx) of a living body, irradiates the finger of the living body with a predetermined measuring light beam, receives a detection light beam derived from the measuring light beam and coming from the inside of the finger, and based on the light-reception result, obtains biological information. Examples of the biological information include a pulse wave, a blood oxygen saturation level, a pulse rate, a cardiac output, and a blood glucose level of the living body.

First, an electrical configuration of the biological information measuring device will be described. As illustrated in FIG. 1, for example, the biological information measuring device LM includes a measuring section 11, a display section 12, a control arithmetic section 13, a charging power supply section 14, an operation section 15, a wireless communication section 16, and a battery section 17.

The measuring section 11 is a circuit that is connected to the control arithmetic section 13 and measures biological information in accordance with a control of the control arithmetic section 13. The measuring section 11 outputs the measurement result to the control arithmetic section 13. The measuring section 11 employs a specific known configuration for biological information to be measured. For example, in the present embodiment, a blood oxygen saturation level (SpO₂) is measured with a photoelectric technique. In this case, the measuring section 11 includes an irradiation (light-emmitting) part 111, a light-receiving part 112, and a current-to-voltage converter (I/V converter) 113.

The irradiation part 111 is a circuit that is connected to the control arithmetic section 13 and applies a measuring light beam with a predetermined wavelength in accordance with a control of the control arithmetic section 13. The light-receiving part 112 is a circuit that is connected to the I/V converter 113, receives a detection light beam, and outputs a light reception result (current obtained by photoelectric conversion of the detection light beam) to the I/V converter 113. The detection light beam is obtained by transmitting the measuring light beam through a part of a living body to be measured, such as a maniphalanx, or by reflecting the measuring light beam on the part (which is a maniphalanx in the embodiment) of the living body. Thus, the irradiation part 111 and the light-receiving part 112 are disposed in an appropriate layout (e.g., opposed layout or separated parallel layout) so as to utilize a transmitted detection light beam or an appropriate layout (e.g., adjacent parallel layout) so as to utilize a reflected detection light beam. In the biological information measuring device LM according to the present embodiment, the irradiation part 111 and the light-receiving part 112 are opposed to each other at a predetermined distance so as to utilize a transmitted detection light beam (opposed layout). The measuring light beam is a light beam having a wavelength at which the biological information, which is a blood oxygen saturation level in the present embodiment, can be detected. More specifically, in the case of detecting a blood oxygen saturation level by using a difference in absorbance in accordance with the wavelength between hemoglobin and oxyhemoglobin, the measuring light beam is, for example, a red light beam or an infrared light beam. The irradiation part 111 includes, for example, an irradiation optical system, a light source such as a light-emitting diode (LED), and a peripheral circuit around the light source such as a driving circuit. The light-receiving part 112 includes a light-receiving optical system, a photoelectric conversion element such as a silicon photodiode, and a peripheral circuit thereof. In this manner, the irradiation part 111 and the light-receiving part 112 constitute an irradiating/light-receiving part that irradiates a part of the living body with a predetermined measuring light beam and receives a detection light beam from the part of the living body derived from the measuring light beam so that the received light beam is used to obtain biological information. The I/V converter 113 is a circuit that is connected to the control arithmetic section 13 and converts a current input from the light-receiving part 112 and generated by photoelectric conversion of a detection light beam in the light-receiving part 112 to a voltage. The obtained voltage is an output from the I/V converter 113 to the control arithmetic section 13 as a measurement result of the measuring section 11.

The control arithmetic section 13 is a circuit that controls each part of the biological information measuring device LM in accordance with the function of the part. Thus, the control arithmetic section 13 controls a measuring operation of the measuring section 11. The control arithmetic section 13 is a circuit that obtains biological information based on the light receiving result of the light-receiving part 112 through the I/V converter 113 in the measuring section 11. The control arithmetic section 13 includes a microcomputer having a central processing unit (CPU), a memory, and peripheral circuits thereof. A first control part 131 and a biological information arithmetic part 132 are functionally configured in the control arithmetic section 13 by executing a program. The first control part 131 controls the parts of the biological information measuring device LM in accordance with the functions thereof. In a case where no continuous signals corresponding to the biological information appear in the light reception result of the light-receiving part 112 in a predetermined time determined by the measuring section 11 beforehand (e.g., the case where the light reception result of the light-receiving part 112 is flat in the range of a noise level), the first control part 131 controls the biological information measuring device LM in a so-called standby state or a sleep state. The first control part 131 stops a measuring operation of the measuring section 11 while the battery section 17 is being charged by the charging power supply section 14. When the charging of the battery section 17 by the charging power supply section 14 is completed, the power supply to the first control part 131 is turned off. The biological information arithmetic part 132 obtains biological information based on a light reception result (photoelectric pulse wave signal) of the light-receiving part 112 in the measuring section 11. A blood oxygen saturation level that is an example of biological information is calculated in the known conventional manner.

The operation section 15 is a section that is connected to the control arithmetic section 13 and inputs a predetermined operation to the biological information measuring device LM, for example, and may be one or more input switches each assigned with a predetermined function, for example. More specifically, in the present embodiment, the operation section 15 is a push-bottom power switch for turning on a power supply to start measurement.

The display section 12 is a section that is connected to the control arithmetic section 13 and displays an operating state of the biological information measuring device LM in accordance with a control of the control arithmetic section 13. The display section 12 includes, for example, an LED in the present embodiment. The LED of the display section 12 displays the operating state of the biological information measuring device LM by changing the color (e.g., red, green, or blue) of emitted light and a lighting state (e.g., continuous lighting or blinking). In the present embodiment, the operating state includes a measuring state in which a measuring operation is being performed and a charging state in which a charging is being performed. The display section 12 may include, for example, a display device such as a liquid crystal display (LCD) or an organic EL display in order to display the type of operation input from the operation section 15 or a measurement result (e.g., a blood oxygen saturation level).

The wireless communication section 16 is a circuit that is connected to the control arithmetic section 13 and transmits a communication signal wirelessly to an external device. The biological information obtained by the control arithmetic section 13 is transmitted by the wireless communication section 16 so that a structure for analyzing and storing the biological information does not need to be installed in the biological information measuring device LM. As a result, a displaying component such as LCD can be omitted, and size reduction, power consumption reduction, and cost reduction of the display device can be achieved. The wireless communication section 16 may transmit the light reception result obtained by the light-receiving part 112 of the measuring section 11 to an external device without change. In this manner, the biological information arithmetic part 132 of the control arithmetic section 13 can be omitted.

The battery section 17 is a circuit that is subjected to charging and discharging of electric power. The battery section 17 is connected to the charging power supply section 14, and components of the biological information measuring device LM that need electric power, such as the irradiation part 111, the display section 12, the control arithmetic section 13, and the wireless communication section 16. The battery section 17 is charged with electric power received by the charging power supply section 14, and supplies the electric power to the components of the biological information measuring device LM that need electric power. The battery section 17 includes a secondary battery (storage battery) and a peripheral circuit thereof, for example. The secondary battery includes, for example, a nickel-cadmium battery, a nickel-metal hydride battery, or a lithium ion battery.

The charging power supply section 14 is a circuit that is connected to the control arithmetic section 13 and the battery section 17 and charges the battery section 17 in a noncontact manner. The charging power supply section 14 includes a charging part 141 and a power receiving part 142, for example. The power receiving part 142 includes a power receiving coil 1421 (see FIG. 2B) that is a coil for generating an electromotive force in a magnetic field by electromagnetic induction and a peripheral circuit 1422 (see FIG. 2B) constituting a resonant circuit in combination with the power receiving coil. The charging part 141 is a circuit that charges the battery section 17 with electric power received by the power receiving part 142.

A structural configuration of the biological information measuring device will be described. As illustrated in FIGS. 2A and 2B, for example, the biological information measuring device LM includes a substantially cuboid upper cover member 181 that is long in a specific direction, and a substantially cuboid lower cover member 182 having substantially the same length and width as those of the upper cover member 181. The upper cover member 181 and the lower cover member 182 face each other and are rotatably coupled to each other at one end by a hinge 183. An unillustrated biasing member is provided around an axis of the hinge 183 so that the upper cover member 181 and the lower cover member 182 are biased in a direction (closing direction) in which the upper cover member 181 and the lower cover member 182 approach each other. An inner surface of the upper cover member 181 is provided with an upper finger receiving member 184 extending in the specific direction from the other end of the upper cover member 181 and having a recessing curved surface along the back of a fingertip of a maniphalanx. An inner surface of the lower cover member 182 is provided with a lower finger receiving member 185 extending in the specific direction from the other end of the lower cover member 182 and having a recessing curved surface along a pad of the fingertip. The upper finger receiving member 184 and the lower finger receiving member 185 correspond to a living body to be measured, i.e., an example of a placement part to which a maniphalanx is placed in the present embodiment. An end of the upper finger receiving member 184 is provided with a restriction strip 186 extending downward, i.e., inward. The living body, which is a maniphalanx in this embodiment, is placed to the placement part constituted by the upper finger receiving member 184 and the lower finger receiving member 185, and the tip of the maniphalanx reaches the restriction strip 186 so that the restriction strip 186 restricts a limit position in entering of a living body (i.e., a maniphalanx in the present embodiment) in the specific direction and restricts (defines) an placement region to which the living body is placed in a case where the living body enters the placement part along the specific direction.

An outer side surface of the upper cover member 181 is provided with the display section 12 and the operation section 15 in such a manner that the display section 12 and the operation section 15 face the outside. The upper cover member 181 encloses the wireless communication section 16 and the battery section 17 disposed in this order from the one end to the other end in the specific direction. An inner surface of the upper cover member 181 is provided with the irradiation part 111 of the measuring section 11 to apply a measuring light beam to the placed living body (maniphalanx in the embodiment). More specifically, the upper finger receiving member 184 includes an irradiation window (illumination window) through which a measuring light beam can pass and which is disposed at an appropriate location.

The lower cover member 182 encloses the power receiving coil 1421 of the charging power supply section 14 and the main board 187 that are disposed in this order from the one end to the other end along the specific direction. The inner surface of the lower cover member 182 is provided with the light-receiving part 112 of the measuring section 11 so that the light-receiving part 112 can receive a detection light beam from the placed living body (maniphalanx in the embodiment). More specifically, the lower finger receiving member 185 is provided with a light-receiving window that is made of a material capable allowing detection light beam to pass and is located at a position facing the irradiation window of the upper finger receiving member 184. Specifically, the location of the irradiation window and the location of the light-receiving window face each other at a predetermined interval. The main board 187 is a board on which a predetermined electrical circuit parts are mounted. On the main board 187, the I/V converter 113 of the measuring section 11, the control arithmetic section 13, the charging part 141 of the charging power supply section 14, and the peripheral circuit 1422 of the power receiving coil 1421, for example, are mounted.

The power receiving coil 1421 is located closer to the one end than the restriction strip 186. Thus, the placement part constituted by the upper finger receiving member 184 and the lower finger receiving member 185 and the power receiving coil 1421 are disposed along the specific direction in the order of the power receiving coil 1421, the restriction strip 186, and the placement region defined by the restriction strip 186.

The biological information measuring device LM described above constitutes a biological information measuring system LS (see FIG. 3) in combination with a charger CM for charging the biological information measuring device LM in a noncontact manner.

As illustrated FIG. 1, for example, the charger CM includes a power transmission part 19, a second control part 20, and a power source part 21. The power transmission part 19 is a circuit that transmits electric power in a noncontact charging manner, and includes a power transmission coil that is a coil for generating a magnetic field and a peripheral circuit constituting a resonant circuit in combination with the power transmission coil, for example. The second control part 20 is a circuit that is connected to the power transmission part 19 and controls an operation of the power transmission part 19. The power source part 21 converts, for example, commercial electric power into an electric power having a predetermined voltage value and a predetermined current value and supplies the electric power to the power transmission part 19 through the second control part 20, and is, for example, an AC adapter.

The power transmission part 19, the second control part 20, and the power source part 21 are enclosed in a substantially cuboid casing 22 that is relatively thin, as illustrated in FIG. 3. The casing 22 has a recessed portion 221 at an appropriate location in a top surface thereof. In charging the biological information measuring device LM, the biological information measuring device LM is enclosed in the recessed portion 221, as illustrated in FIG. 3.

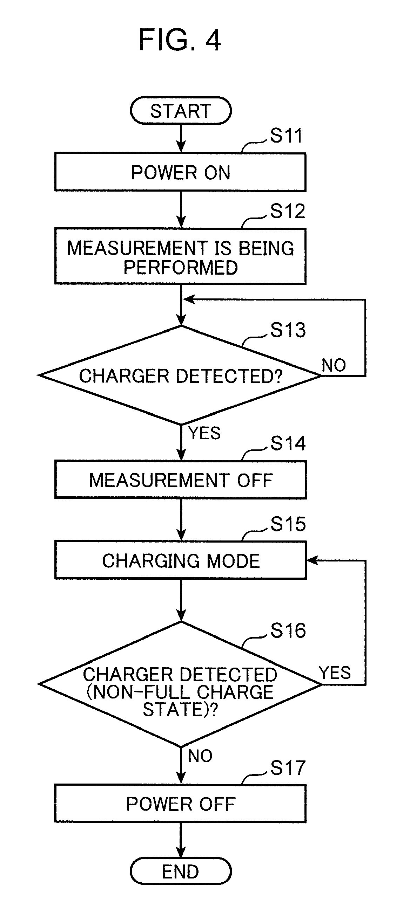

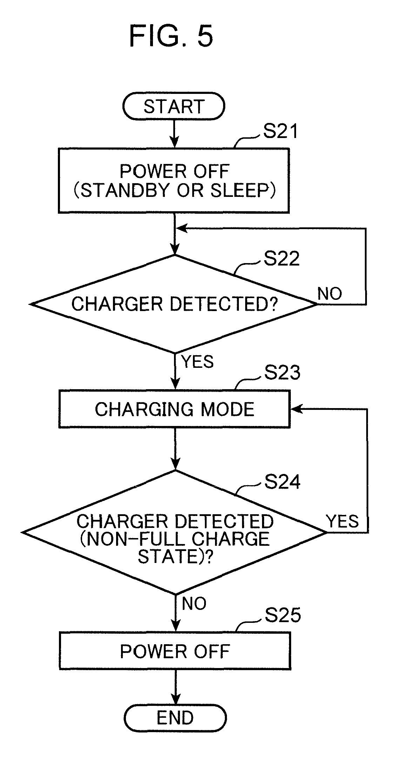

Next, an operation of the present embodiment will be described. FIG. 4 is a flowchart showing an operation of the biological information measuring device in a case where charging starts while measurement is performed. FIG. 5 is a flowchart showing an operation of the biological information measuring device in a case where charging starts while no measurement is performed.

First, an operation in the case where charging starts while a measurement is being performed will be described. In a measurement of biological information, the biological information measuring device LM grips or clips a maniphalanx, and is put on the maniphalanx in the configuration described above. In FIG. 4, when the operation section 15 is operated by a user so that the biological information measuring device LM is powered on (S11), the control arithmetic section 13 initializes necessary parts, and a measurement of biological information is started by the measuring section 11 and the control arithmetic section 13 so that the biological information measuring device LM comes into a measurement state (S12). In this measurement state, the display section 12 continuously illuminates green, which indicates a measuring state, for example.

In such measurement, a connection with the charger CM is checked repeatedly by the control arithmetic section 13 and the charging power supply section 14 (No in S13) until the charger CM is detected. When the connection with the charger CM is detected (Yes in S13), the first control part 131 of the control arithmetic section 13 forcedly stops a measuring operation of the measuring section 11 (S 14). In this manner, one of a measuring step or process of measuring biological information or a charging step or process of charging electric power in a noncontact manner is selectively executed.

The checking of the charger CM to determine whether the charger CM is connected or not is carried out by determining whether the power receiving coil 1421 generates an electromotive force or not, for example. If the power receiving coil 1421 generates an electromotive force, the control arithmetic section 13 determines that the charger CM is connected. On the other hand, if the power receiving coil 1421 does not generate an electromotive force, the control arithmetic section 13 determines that the charger CM is not connected. For example, the checking of the charger CM may be performed by intercommunication between the biological information measuring device LM and the charger CM. The intercommunication function is provided in order to prevent the charger CM from transmitting electric power when other object than the biological information measuring device LM, such as a clip, lies in the charger CM at a transmission side. The intercommunication can be performed by providing the second control part 20 with a communication function so that the power transmission coil of the charger CM is used as an antenna and by providing the control arithmetic section 13 with a communication function so that the power receiving coil 1421 of the biological information measuring device LM is used as an antenna. In a case where a signal is received from the charger CM, the control arithmetic section 13 determines that the charger CM is connected. On the other hand, in a case where no signal is received from the charger CM, the control arithmetic section 13 determines that the charger CM is not connected.

As described above, when the measuring operation is stopped at step S14, the control arithmetic section 13 and the charging power supply section 14 start charging of the battery section 17 so that the biological information measuring device LM is in the process of being charged (S15). In this charging state or charging mode, the display section 12 continuously illuminates red, which indicates a charging state, for example. The user can recognize that the measuring operation is suspended owing to the fact that the display section 12 is changed from the indication of the measuring state to the indication of the charging state, and the biological information measuring device LM can guide the user to avoid using the biological information measuring device LM.

In the charging state, the charger CM is repeatedly detected by the control arithmetic section 13 and the charging power supply section 14 (Yes in S16). When the charger CM is not detect any more (No in S16), the first control part 131 of the control arithmetic section 13 renders the device to be powered off (S 17), and the operation is finished.

In step S16, it may be appreciated that instead of the checking of the charger CM or in addition to the checking of the charger CM, the charging power supply section 14 is further provided with a full-charge detection part for detecting a full charge state of the battery section 17 or a residual power amount detection part for detecting a residual power amount of the battery section 17 to repeatedly check the charged state of the battery section 17 by the full-charge detection part or the residual power amount detection part, and the flow is allowed to proceed to step S 17 when the full-charge state of the battery section 17 is detected (Yes in S16).

Next, an operation in the case where charging starts while a measurement is not performed will be described. In FIG. 5, when the biological information measuring device LM is powered off in the standby state or the sleep state (S21), a connection with the charger CM is checked repeatedly by the control arithmetic section 13 and the charging power supply section 14 (No in S22) until the charger CM is detected. When the connection with the charger CM is detected (Yes in S22), the control arithmetic section 13 and the charging power supply section 14 start charging of the battery section 17. In other words, the biological information measuring device LM comes into the charging state (S23). In this charging state (charging mode), as described above, the display section 12 continuously illuminates red, which indicates the charging state, for example. Thus, the user can recognize that the biological information measuring device LM is in the charging state, and the biological information measuring device LM can guide the user to avoid using the biological information measuring device LM. In the charging state, the first control part 131 controls the measuring section 11 in such a manner that the measurement of the measuring section 11 is forcedly suspended even when the user operates the operation section 15.

In the charging state, the charger CM is repeatedly detected by the control arithmetic section 13 and the charging power supply section 14 (Yes in S24). When the charger CM is not detected any more (No in S24), the first control part 131 of the control arithmetic section 13 renders the device to be powered off (S25), and the operation is finished.

In step S24, it may be appreciated that instead of the checking of the charger CM or in addition to the checking of the charger CM, in a similar manner to step S16, the charging power supply section 14 is further provided with a full-charge detection part for detecting a full charge state of the battery section 17 or a residual power amount detection part for detecting a residual power amount of the battery section 17 to repeatedly check the charged state of the battery section 17 (No in S24), and the flow is allowed to proceed to step S25 when the full-charge state of the battery section 17 is detected (Yes in S24).

As described above, in the biological information measuring device LM and the biological information measuring method using the biological information measuring device LM, one of the measuring step and the charging step is selectively executed, and the measuring operation of the measuring section 11 is suspended while the battery section 17 is charged by the charging power supply section 14. Thus, the user can be guided to avoid using the biological information measuring device LM. Accordingly, the biological information measuring device LM and the biological information measuring method using the biological information measuring device LM can satisfy predetermined safety standards concerning the biological information measuring device LM.

As described above, in the biological information measuring device LM and the biological information measuring method using the biological information measuring device LM, the measurement operation is suspended during charging. Accordingly, it will be seen that the user does not use the biological information measuring device immediately even when the charging is completed and a measurement can be performed. It can be understood that the battery section 17 wastefully discharges electric power, if the power supply remains on after the charging is completed but the measuring section 11 performs no significant measuring operation. However, in the biological information measuring device LM and the biological information measuring method using this device according to the present embodiment, the first control part renders the device to be powered off when the charging of the battery section 17 by the charging power supply section 14 is completed. Thus, wasteful discharge of the battery section 17 can be suppressed.

In the noncontact charging, heat is likely to generate mainly in the power receiving coil 1421 and a resonant circuit thereof (peripheral circuit 1422). In the biological information measuring device LM according to the present embodiment, the placement part constituted by the upper finger receiving member 184 and the lower finger receiving member 185, and the power receiving coil 1421 are arranged in such a manner that the power receiving coil 1421, the restriction strip 186, and the placement region lie along a predetermined direction. As compared with a configuration that a placement region and a power receiving coil region overlap each other, the placement region of the placement part has a reduced temperature increase caused by heat generated by the power receiving coil. Thus, the biological information measuring device LM according to the present embodiment makes it possible to start measurement quickly after the charging is completed.

In the embodiment described above, the charging power supply section 14 may be provided with a residual power amount detection part for detecting a residual power amount of the battery section 17. In this configuration, if a residual power amount detected by the residual power amount detection part is greater than or equal to a predetermined threshold value when the charging power supply section 14 starts charging the battery section 17, the first control part 131 stops starting of charging of the battery section 17 by the charging power supply section 14 to allow a measurement operation of the measuring section 11 to be executed. To detect a residual power amount, a known technique may be executed. For example, measurement of an inter-terminal voltage of the battery section 17, or measurement of a difference between an inflow current and an outflow current in the battery section 17 is executed. The predetermined threshold value is one of 50%, 40%, or 30%, for example. The biological information measuring device LM includes the first control part 131 that stops starting of charging of the battery section 17 by the charging power supply section 14 and allows a measurement operation of the measuring section 11 when a residual power amount detected by the residual power amount detection part is greater than or equal to the predetermined threshold value in the process of charging the battery section 17 by the charging power supply section 14. Thus, in a case where the charging of the battery section 17 is not necessarily required, a measurement of the measuring section 11 can be performed in priority to the charging. In particular, to obtain more information on a living body, it is preferable to monitor biological information continuously and persistently. The biological information measuring device LM which monitors biological information continuously and persistently can be seen to be preferable in this point.

In the present specification, techniques in various aspects have been disclosed, and major ones of these techniques are summarized below.

A biological information measuring device according to an aspect comprises: a measuring section that measures biological information; a battery section that is subjected to charge and discharge of electric power; a charging power supply section that supplies charging power to the battery section to execute charging of the battery section in a noncontact manner; and a control section that controls a measuring operation of the measuring section. The control section suspends the measuring operation of the measuring section during the charging of the battery section by the charging power supply section.

In the biological information measuring device, since the measuring operation of the measuring section is suspended during the charging of the battery section by the charging power supply section, a user can be guided to avoid using the biological information measuring device. Thus, the biological information measuring device can satisfy predetermined safety standards concerning biological information measuring devices.

According to another aspect, in the biological information measuring device described above, the charging power supply section includes a residual power amount detection part that detects a residual power amount of the battery section, and the control section suspends the charging of the battery section by the charging power supply section and allows the measuring operation of the measuring section if the residual power amount detected by the residual power amount detection part is greater than or equal to a predetermined threshold value when starting the charging of the battery section by the charging power supply section.

The biological information measuring device includes the control section that suspends the charging of the battery section by the charging power supply section and allows the measuring operation of the measuring section if the residual power amount detected by the residual power amount detection part is greater than or equal to the predetermined threshold value when starting the charging of the battery section by the charging power supply section. Thus, when charging of the battery section is not necessarily required, measurement of the measuring section is allowed in priority to the charging. In particular, to obtain more information on a living body, it is preferable to monitor biological information continuously and persistently. The biological information measuring device can monitor biological information continuously and persistently, and thus, is preferable in this respect.

According to another aspect, in any of the biological information measuring devices described above, the control section renders the device to be powered off when the charging of the battery section by the charging power supply section is completed.

In a biological information measuring device which suspends a measuring operation during charging, the user can be seen not to use the biological information measuring device immediately even when the charging is completed and a measurement can be performed, and it will be seen that the battery section wastefully discharges electric power, if the power supply remains on after the charging is completed but the measuring section performs no significant measuring operation. However, since the biological information measuring device described above includes the control section that renders the device to be powered off when the charging of the battery section by the charging power supply section is completed, wasteful discharge of the battery section can be suppressed.

According to another aspect, in any of the biological information measuring devices described above, the measuring section includes an placement part to which a living body to be measured is placed, the charging power supply section includes a power receiving coil that generates an electromotive force in a magnetic field owing to an electromagnetic induction, the placement part includes a restriction portion that restricts an placement region to which the living body is placed, and the placement part and the power receiving coil are disposed in such a manner that the power receiving coil, the restriction portion, and the placement region lie along a predetermined direction.

In the case of noncontact charging, heat is likely to generate mainly in a power receiving coil or a resonant circuit thereof. In the biological information measuring device described above, the placement part and the power receiving coil are arranged in such a manner that the power receiving coil, the restriction portion, and the placement region lie along the predetermined direction. As compared to a configuration that an placement region and a power receiving coil region overlap each other, the placement region of the placement part has a reduced temperature increase caused by heat generated by the power receiving coil. Thus, the biological information measuring device makes it possible to start measurement immediately after the charging is completed.

According to another aspect, a biological information measuring method using a biological information measuring device comprises a measuring step of measuring biological information and a charging step of performing charging of electric power in a noncontact manner, one of the measuring step and the charging step is selectively executed.

The biological information measuring method is applied to a biological information measuring device to execute one of the measuring step and the charging step selectively. Thus, in the biological information measuring method, since the measuring step is suspended while the charging step is performed, the user can be guided to avoid using the biological information measuring device. As a result, the biological information measuring method can satisfy predetermined safety standards concerning biological information measuring devices.

This application is based on Japanese Patent Application No. 2014-241444 filed in Japan Patent Office on November 28, 2014, the contents of which are hereby incorporated by reference.

Although the present invention has been fully described by way of embodiment with reference to the accompanying drawings, it is to be understood that various changes and modifications will be apparent to those skilled in the art. Therefore, unless otherwise such changes and modifications depart from the scope of the present invention defined in claims, they should be construed as being included therein.

## Claims

1. A biological information measuring device, comprising
a measuring section that measures biological information;
a battery section that is subjected to charge and discharge of electric power;
a charging power supply section that supplies charging power to the battery section to execute charging of the battery section in a noncontact manner; and
a control section that controls a measuring operation of the measuring section, the control section suspending the measuring operation of the measuring section during the charging of the battery section by the charging power supply section.

2. A biological information measuring device according to claim 1, wherein
the charging power supply section includes a residual power amount detection part that detects a residual power amount of the battery section, and
the control section suspends the charging of the battery section by the charging power supply section and allows the measuring operation of the measuring section if the residual power amount detected by the residual power amount detection part is greater than or equal to a predetermined threshold value when starting the charging of the battery section by the charging power supply section,

3. A biological information measuring device according to claim 1 or 2, wherein
the control section renders the device to be powered off when the charging of the battery section by the charging power supply section is completed.

4. A biological information measuring device according to any one of claim 1 to 3, wherein
the measuring section includes an placement part to which a living body to be measured is placed,
the charging power supply section includes a power receiving coil that generates an electromotive force in a magnetic field owing to an electromagnetic induction,
the placement part includes a restriction portion that restricts an placement region to which the living body is placed, and
the placement part and the power receiving coil are disposed in such a manner that the power receiving coil, the restriction portion, and the placement region lie along a predetermined direction.

5. A biological information measuring method using a biological information measuring device, the method comprising:
a measuring step of measuring biological information; and
a charging step of performing charging of electric power in a noncontact manner, wherein
one of the measuring step and the charging step is selectively executed.
